(19) Europäisches Patentamt
European Patent Office
Office européen des brevets

(11) **EP 2 383 347 B1**

(12) **EUROPEAN PATENT SPECIFICATION**

(45) Date of publication and mention
of the grant of the patent:
**11.06.2014  Bulletin 2014/24**

(51) Int Cl.:
***C12Q 1/68*** (2006.01)

(21) Application number: **11164214.6**

(22) Date of filing: **28.04.2011**

(54) **Method for detecting mutation in Exon 12 of JAK2 gene, and nucleic acid probe and kit therefor**

Verfahren zur Detektion einer Mutation in Exon 12 vom JAK2-Gen sowie Nukleinsäuresonde und Kit dafür

Procédé pour détecter la mutation au niveau d'Exon 12 du gène JAK2, sonde d'acide nucléique et kit correspondant

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2010  JP 2010105457**

(43) Date of publication of application:
**02.11.2011  Bulletin 2011/44**

(73) Proprietor: **ARKRAY, Inc.**
**Kyoto-shi, Kyoto 601-8045 (JP)**

(72) Inventor: **Komori, Mariko**
**Kyoto-shi**
**Kyoto 602-0008 (JP)**

(74) Representative: **Jones, Elizabeth Louise**
**Dehns**
**St Bride's House**
**10 Salisbury Square**
**London**
**EC4Y 8JD (GB)**

(56) References cited:
EP-A1- 2 119 796      EP-A2- 1 362 928
WO-A2-2007/106899     JP-A- 2001 286 300
JP-A- 2002 119 291    JP-A- 2009 278 982

• VALERIE UGO ET AL: "Interlaboratory Development and Validation of a HRM Method Applied to the Detection of JAK2 Exon 12 Mutations in Polycythemia Vera Patients", PLOS ONE, vol. 5, no. 1, 1 January 2010 (2010-01-01) , pages E8893-E8893, XP55006949, ISSN: 1932-6203, DOI: 10.1371/journal.pone.0008893

• A. V. JONES ET AL: "Rapid identification of JAK2 exon 12 mutations using high resolution melting analysis", HAEMATOLOGICA, vol. 93, no. 10, 1 October 2008 (2008-10-01), pages 1560-1564, XP55006957, ISSN: 0390-6078, DOI: 10.3324/haematol.12883

• INMACULADA RAPADO ET AL: "High Resolution Melting Analysis for JAK2 Exon 14 and Exon 12 Mutations", THE JOURNAL OF MOLECULAR DIAGNOSTICS, vol. 11, no. 2, 1 March 2009 (2009-03-01), pages 155-161, XP55006960, ISSN: 1525-1578, DOI: 10.2353/jmoldx.2009.080110

• INMACULADA RAPADO ET AL: "Validity test study of JAK2 V617F and allele burden quantification in the diagnosis of myeloproliferative diseases", ANNALS OF HEMATOLOGY, SPRINGER, BERLIN, DE, vol. 87, no. 9, 25 June 2008 (2008-06-25), pages 741-749, XP019625424, ISSN: 1432-0584

• TANAKA R ET AL: "Fully automated and super-rapid system for the detection of JAK2V617F mutation", LEUKEMIA RESEARCH, NEW YORK, NY, US, vol. 32, no. 9, 1 September 2008 (2008-09-01), pages 1462-1467, XP022659315, ISSN: 0145-2126, DOI: 10.1016/J.LEUKRES. 2007.12.019 [retrieved on 2008-03-06]

**(Cont. next page)**

Remarks:
The file contains technical information submitted after the application was filed and not included in this specification

Remarks:
The file contains technical information submitted after the application was filed and not included in this specification

SEQUENCE LISTING

<110> Arkray, Inc.

<120> Method for Detecting Mutation in Exon 12 of JAK2 Gene, and Nucleic Acid Probe and Kit Therefor

<130> 42.13.109330

<150> JP2010-105457
<151> 2010-4-30

<160> 14

<170> Patentin version 3.5

<210> 1
<211> 240
<212> DNA
<213> Homo sapiens

<400> 1

```
atatgtattt tattttttca gataaatcaa accttctagt cttcagaacg aatggtgttt        60

ctgatgtacc aacctcacca acattacaga ggcctactca tatgaaccaa atggtgtttc       120

acaaaatcag aaatgaagat ttgatatttg taagtcatta gatactcatt actgtctttt       180

ttgtcctttt aaaacaacat ctgttttctt gatttacatt catgtgacat tggaattatt       240
```

<210> 2
<211> 31
<212> DNA
<213> Artificial Sequence

<220>
<223> forward primer

<400> 2
ctgatgtacc aacctcacca acattacaga g        31

<210> 3
<211> 35
<212> DNA
<213> Artificial Sequence

<220>
<223> reverse primer

<400> 3

aaggacaaaa aagacagtaa tgagtatcta atgac          35

<210> 4
<211> 19
<212> DNA
<213> Artificial Sequence

<220>
<223> del6inL probe

<400> 4
cttcatttct gattaacac          19

<210> 5
<211> 32
<212> DNA
<213> Artificial Sequence

<220>
<223> WT probe 1

<400> 5
caaatcttca tttctgattt tgtgaaacac ca          32

<210> 6
<211> 27
<212> DNA
<213> Artificial Sequence

<220>
<223> WT probe 2

<400> 6
atcttcattt ctgattttgt gaaacac          27

<210> 7
<211> 59
<212> DNA
<213> Homo sapiens

<400> 7
atgagtatct aatgacttac aaatatcaaa tcttcatttc tgattttgtg aaacaccat          59

<210> 8
<211> 59
<212> DNA
<213> Homo sapiens

<400> 8
atgagtatct aatgacttac aaatatcaaa tcttcatttc tgattaagtg aaacaccat          59

<210> 9
<211> 59
<212> DNA
<213> Homo sapiens

<400> 9
atgagtatct aatgacttac aaatatcaaa tcttcatttc tgattaattg aaacaccat          59

<210> 10
<211> 53
<212> DNA
<213> Homo sapiens

<400> 10
atgagtatct aatgacttac aaatatcaaa tctctgattt tgtgaaacac cat        53

<210> 11
<211> 53
<212> DNA
<213> Homo sapiens

<400> 11
atgagtatct aatgacttac aaatatcaaa tcttcatttc tgattaacac cat        53

<210> 12
<211> 30
<212> DNA
<213> Homo sapiens

<400> 12
tggtgtttca caaaatcaga aatgaagatt        30

<210> 13
<211> 27
<212> DNA
<213> Homo sapiens

<400> 13
tggtgtttct aatcagaaat gaagatt        27

<210> 14
<211> 18
<212> DNA
<213> Artificial Sequence

<220>
<223> WT probe 3

<400> 14
cagaaatgaa gatttgat        18

**Description**

TECHNICAL FIELD

The present invention relates to a method for detecting a mutation in exon 12 of the JAK2 gene, and a nucleic acid probe and a kit therefor.

BACKGROUND OF THE INVENTION

A mutation in exon 12 of the JAK2 gene is observed in patients suffering from polycythemia vera (PV), which is a chronic myeloproliferative disorder, and presence/absence of the mutation is one of the criteria for polycythemia vera (BLOOD, 30 JULY 2009 VOLUME 114, NUMBER 5).

Examples of the method to detect the mutation in exon 12 of the JAK2 gene include a method wherein the detection is carried out by direct sequencing (Haematologica 2007 Dec;92(12):1717-8) and a method wherein the detection is carried out by the PCR-RFLP method or the allele-specific PCR method (AS-PCR method) (N Engl J Med. 2007 Feb 1;356(5):459-68).

The method in Haematologica 2007 Dec;92(12):1717-8 is laborious and costly since it requires, for example, a process in which DNA is extracted and purified, followed by carrying out PCR, a sequencing reaction and electrophoresis in a sequencer. Further, since the amplification product needs to be processed after the PCR, there are problems such as the risk of contamination of the amplification product in the subsequent reaction system, difficulty of automation, and inability to test multiple nucleic acid sequences at the same time.

The PCR-RFLP method in N Engl J Med. 2007 Feb 1;356(5):459-68 is laborious and costly since it requires, for example, a process in which DNA is extracted and purified, followed by carrying out PCR, restriction enzyme treatment of the amplification product and electrophoresis. Further, since the amplification product needs to be processed after the PCR, there are problems such as the risk of contamination of the amplification product in the subsequent reaction system, difficulty of automation, and impossibility of testing multiple nucleic acid sequences at the same time.

The AS-PCR method in N Engl J Med. 2007 Feb 1;356(5):459-68 is a method in which PCR is carried out with primers for sequence-specific amplification, followed by detecting presence/absence of the sequence of interest based on presence/absence of amplification. Since, in this method, primers specific to the respective sequences are used and the reaction is carried out for each of the primers, in cases where there are many mutations to be investigated, the number of reactions is large, which is laborious and costly. Further, the AS-PCR method cannot detect unknown mutations, which is problematic.

On the other hand, there is a known method in which the region containing a mutation is amplified by PCR and melting curve analysis is carried out using a nucleic acid probe labeled with a fluorescent dye, followed by analyzing the mutation based on the result of the melting curve analysis (JP 2001-286300 A and JP 2002-119291 A). However, this literature only teaches that the probe is designed such that, when a quenching probe labeled at its end with a fluorescent dye is hybridized to the target nucleic acid, the multiple nucleotide pairs of the probe-nucleic acid hybrid form at least one GC pair at its end.

Haematologica 2009; 94(3):414-418 describes that cDNA from peripheral blood or bone marrow was subjected to melting curve analysis with 2 probes, and 4 novel mutations (D544-L545del, H538DK539LI540S, H538-K539del and V536-F547dup), in addition to known 5 mutations (E543-D544del, F537-K539delinsL, H538QK539L, K539L and N542-E543del), in exon 12 of the JAK2 gene could be detected.

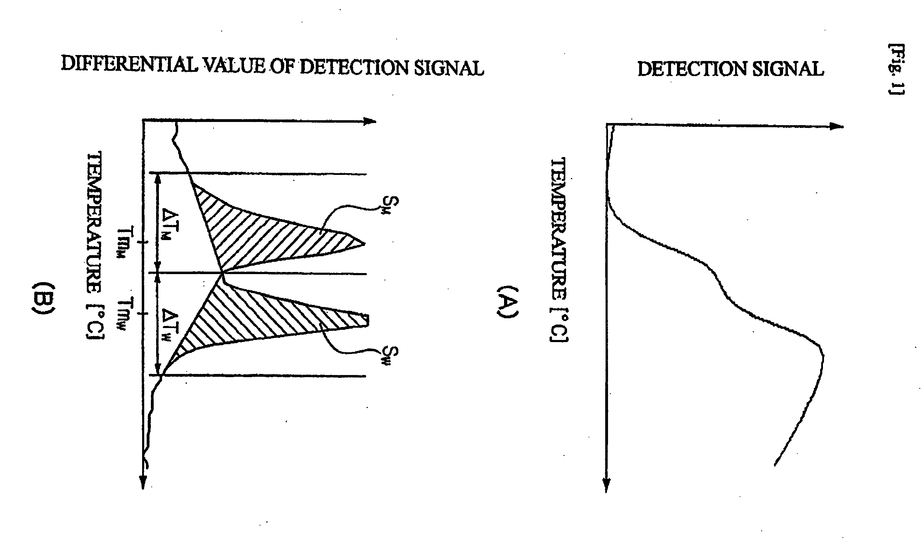

Although the 2 probes in Haematologica 2009; 94(3):414-418 are those to be used for melting curve analysis, their ends are not G or C that is fluorescently labeled. Further, Haematologica 2009; 94(3):414-418 shows that the H538QK539L mutation could be detected (Fig. 1), but the sensitivity was not sufficient, and no data is shown about the fact that the other 8 mutations can be detected. Further, in Haematologica 2009; 94(3):414-418, only cDNA from peripheral blood or bone marrow was used, and there is no teaching that the test can be directly carried out for whole blood.

Ugo et al. (2010, PLOS One, 5;e8893,1-6) describes a High Resolution DNA Melting curve analysis method for detecting JAK2 exon 12 mutations and validation of said method in different laboratories and on different instruments.

Jones et al. (2008, Haematologica, 93(10);1560-1564) describes high resolution melting analysis for detecting JAK2 exon 12 mutations.

Rapado et al. (2009, The Journal of Molecular Diagnostics, 11;155-161) describes high resolution melting analysis for screening of JAK2 exon 14 and exon 12 mutations.

JP 2009278982 describes a method for determining the presence/absence of the G1849T mutant in the JAK2 gene in a nucleic acid specimen using melting curve analysis and a fluorescence resonance energy transfer (FRET) hybridization probe.

WO 2007/106899 describes a system and methods for identifying JAK2-specific polynucleotide sequences in a biological sample. Also described are oligonucleotide primer and probe compositions for detecting JAK2 mutations, in particular the JAK2 V617F mutation.

EP 2119796 describes a probe for detecting a mutation in the JAK2 gene associated with chronic myloproliferative disorder (CMPD) and a method of detecting the mutation using said probe.

Radado et al. (2008, Annals of Hematology, 87;741-749) describes real-time PCR methods to detect the JAK2 V617F mutation using hybridization probes and peptide nucleic acid (PNA) probes specific for wild-type allele and ASO primers for the JAK2 V617F mutant allele and a MGB TaqMan probe.

EP 1362928 describes methods for analyzing a target nucleic acid by incorporating a nucleic acid to which a fluorescent label is attached into at least one strand of the target nucleic acid and monitoring the change in fluorescence emission resulting from dissociation of the labeled strand of the amplification product from its complementary strand.

Tanaka et al. (2008, Leukemia research, 32;1462-1467) describes an automated JAK2 V617F detection system utilizing a JAK2 V617F-specific guanine quenching probe.

SUMMARY OF THE INVENTION

The present invention aims to identify a quenching probe which is effective for detecting a mutation in exon 12 of the JAK2 gene and thereby to provide a method for detecting the mutation in exon 12 of the JAK2 gene and a kit therefor.

The present inventors discovered that, by designing a quenching probe based on a particular region containing a mutation in exon 12 of the JAK2 gene, the mutation can be detected by melting curve analysis using the quenching probe, thereby completing the present invention.

That is, the present invention is as follows.

(1) A probe for detecting a polymorphism in exon 12 of the JAK2 gene, said probe comprising at least one of the oligonucleotides in (a) to (c) below:

(a) an oligonucleotide having a nucleotide sequence homologous to the sequence complementary to a nucleotide sequence having a length of 19 to 50 nucleotides containing the nucleotides 114 to 132 of SEQ ID NO: 1, wherein said oligonucleotide has the nucleotide corresponding to the nucleotide 114 labeled with a fluorescent dye at the first, second or third position from the 3' end;

(b) an oligonucleotide having a nucleotide sequence homologous to the sequence complementary to a nucleotide sequence having a length of 22 to 50 nucleotides containing the nucleotides 122 to 143 of SEQ ID NO:1 wherein said oligonucleotide has the nucleotide corresponding to the nucleotide 143 labeled with a fluorescent dye at the first, second or third position from the 5' end; and

(c) an oligonucleotide having a nucleotide sequence homologous to a nucleotide sequence having a length of 10 to 50 nucleotides containing the nucleotides 41 to 50 of SEQ ID NO: 11, wherein said oligonucleotide shown in (c) has the nucleotide corresponding to the nucleotide 50 labeled with a fluorescent dye at the first, second or third position from the 3' end.

(2) The probe according to (1), wherein
said oligonucleotide shown in (a) has the nucleotide corresponding to the nucleotide 114 labeled with a fluorescent dye at the 3' end;
said oligonucleotide shown in (b) has the nucleotide corresponding to the nucleotide 143 at the 5' end; and/or
said oligonucleotide shown in (c) has the nucleotide corresponding to the nucleotide 50 at the 3' end.

(3) The probe according to (1) or (2), wherein the fluorescence intensity decreases or increases when said oligonucleotide is hybridized with the target sequence, compared with when said oligonucleotide is not hybridized with the target sequence.

(4) The probe according to (3), wherein the fluorescence intensity decreases when said oligonucleotide is hybridized with the target sequence.

(5) The probe according to any one of (1) to (4), wherein
said oligonucleotide shown in (a) has a length of 24 to 30 nucleotides;
said oligonucleotide shown in (b) has a length of 29 to 35 nucleotides; and/or
said oligonucleotide shown in (c) has a length of 16 to 22 nucleotides.

(6) The probe according to any one of (1) to (4), wherein
said oligonucleotide shown in (a) has the nucleotide sequence shown in SEQ ID NO:6;
said oligonucleotide shown in (b) has the nucleotide sequence shown in SEQ ID NO:5; and/or
said oligonucleotide shown in (c) has the nucleotide sequence shown in SEQ ID NO:4.

(7) The probe according to any one of (1) to (6), wherein said polymorphism in exon 12 of the JAK2 gene is at least one mutation selected from the group consisting of K539L(sub1), H538QK539L(sub2), N542-E543del(de16) and F537-K539delinsL(del6inL).

(8) A method for detecting a mutation in a nucleic acid comprising a polymorphic site in exon 12 of the JAK2 gene, comprising the step of carrying out melting curve analysis using a nucleic acid probe labeled with a fluorescent dye for measuring fluorescence from the fluorescent dye, and detecting the mutation based on the result of the melting curve analysis, wherein the nucleic acid probe is the probe according to any one of (1) to (7).

(9) The method according to (8), further comprising the step of obtaining the nucleic acid comprising a polymorphism by amplifying the region containing the polymorphic site in the nucleic acid contained in a sample.

(10) The method according to (9), wherein the amplification is carried out by a method using a DNA polymerase.

(11) The method according to (10), wherein the amplification is carried out in the presence of said probe.

(12) A kit for detecting a polymorphism in exon 12 of the JAK2 gene, comprising the probe according to any one of (1) to (7).

(13) The kit according to (12), further comprising a primer for amplifying the region containing the polymorphic site in exon 12 of the JAK2 gene by a method using a DNA polymerase.

By adding the probe of the present invention in a gene amplification system and only carrying out melting curve analysis (Tm analysis) after PCR reaction, typing of multiple gene variants is possible. Further, since whole blood or an oral mucosa suspension can be directly tested, labour and cost can be reduced.

By using the method of the present invention, the amplification product does not need to be handled after PCR is carried out, so that there is hardly any risk of contamination. Further, since the method of the present invention has a simple mechanism, it can be easily automated.

The probe of the present invention has high specificity and high detection sensitivity. Further, as long as the mutation to be detected is located within the range of the probe, an unknown mutation can also be detected.

Since, by using the probe of the present invention, presence/absence of a mutation in exon 12 of the JAK2 gene can be detected, diagnosis of chronic myeloproliferative disorders and polycythemia vera is possible together with diagnostic items such as the increase of hemoglobin value and erythrocyte amount.

BRIEF DESCRIPTION OF THE DRAWINGS

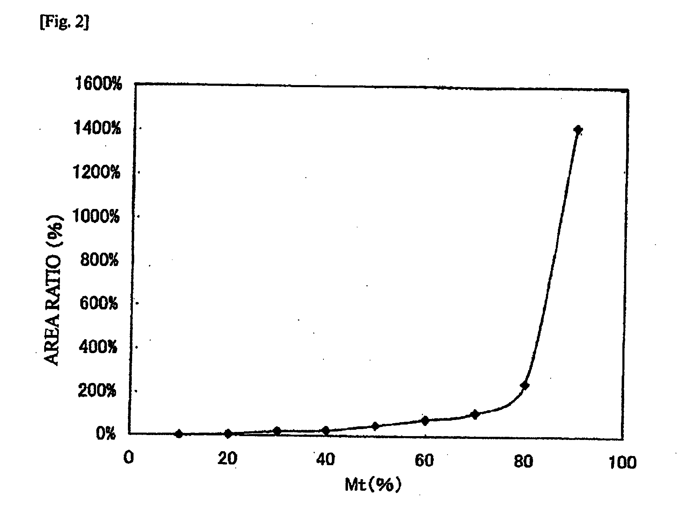

Fig. 1 (A) shows a melting curve for a nucleic acid mixture. Fig. 1 (B) shows an example of the differential melting curve. Fig. 2 shows an example of the calibration curve.

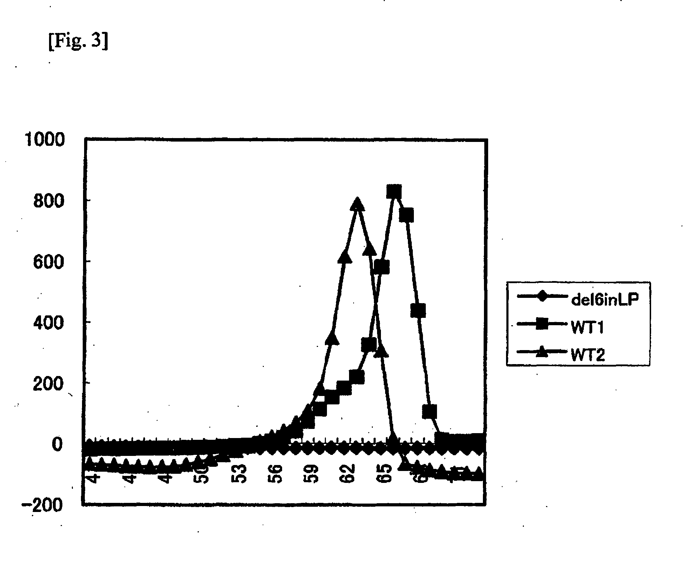

Fig. 3 shows changes in the amount of change in the fluorescence intensity per unit time (d the amount of increase in the fluorescence intensity/t) in Tm analysis for Wt 100% in Example 1. The ordinate indicates the amount of change in the fluorescence intensity per unit time (d the amount of increase in the fluorescence intensity/t), and the abscissa indicates the temperature (°C).

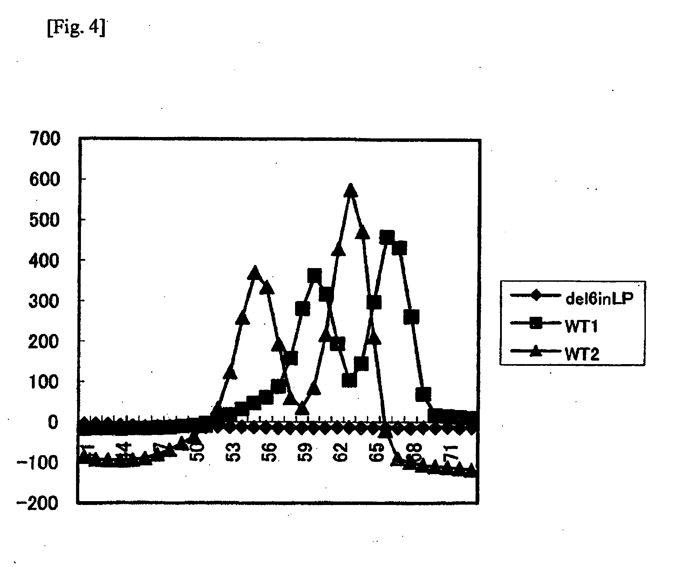

Fig. 4 shows changes in the amount of change in the fluorescence intensity per unit time (d the amount of increase in the fluorescence intensity/t) in Tm analysis for sub 1 50% in Example 1. The ordinate indicates the amount of change in the fluorescence intensity per unit time (d the amount of increase in the fluorescence intensity/t), and the abscissa indicates the temperature (°C).

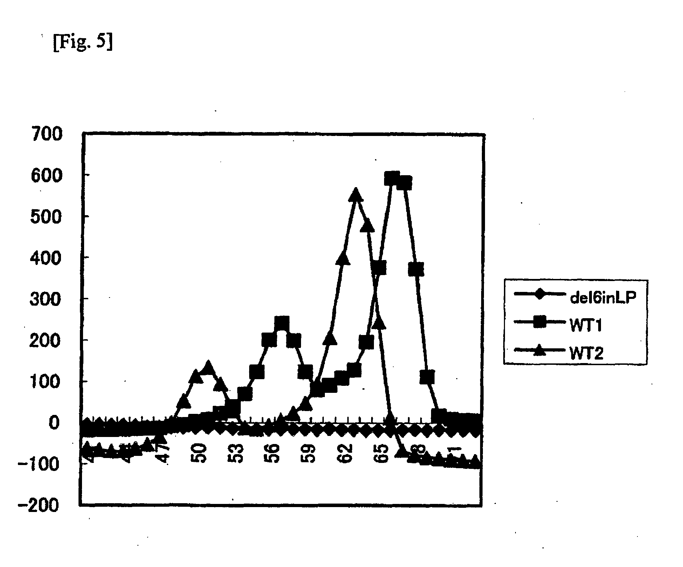

Fig. 5 shows changes in the amount of change in the fluorescence intensity per unit time (d the amount of increase in the fluorescence intensity/t) in Tm analysis for sub2 50% in Example 1. The ordinate indicates the amount of change in the fluorescence intensity per unit time (d the amount of increase in the fluorescence intensity/t), and the abscissa indicates the temperature (°C).

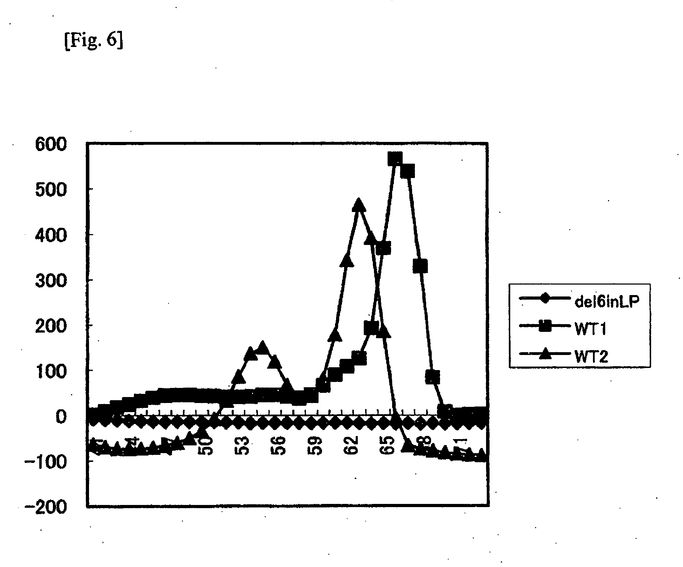

Fig. 6 shows changes in the amount of change in the fluorescence intensity per unit time (d the amount of increase in the fluorescence intensity/t) in Tm analysis for del6 50% in Example 1. The ordinate indicates the amount of change in the fluorescence intensity per unit time (d the amount of increase in the fluorescence intensity/t), and the abscissa indicates the temperature (°C).

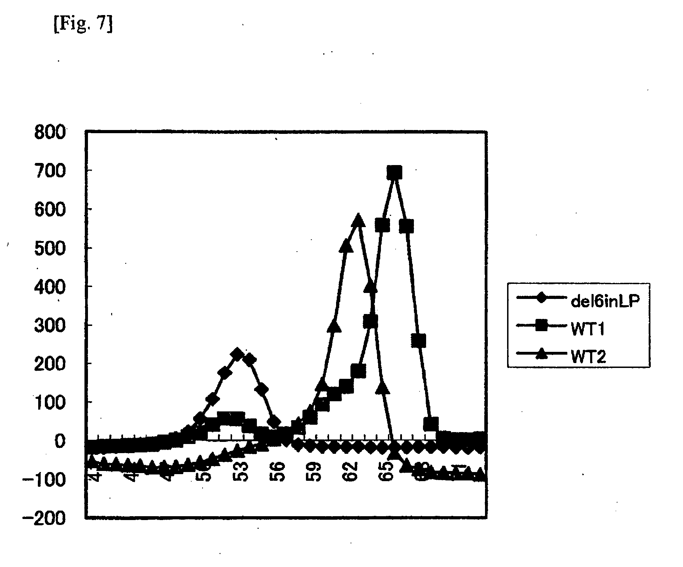

Fig. 7 shows changes in the amount of change in the fluorescence intensity per unit time (d the amount of increase in the fluorescence intensity/t) in Tm analysis for del6inL 50% in Example 1. The ordinate indicates the amount of change in the fluorescence intensity per unit time (d the amount of increase in the fluorescence intensity/t), and the abscissa indicates the temperature (°C).

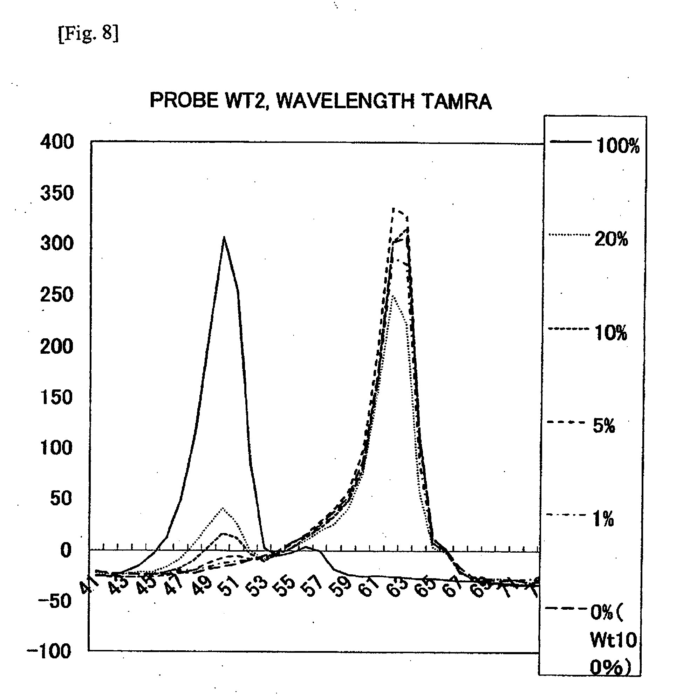

Fig. 8 shows changes in the amount of change in the fluorescence intensity per unit time (d the amount of increase in the fluorescence intensity/t) in Tm analysis for samples in which the mutant plasmid H538QK539L(sub2) is contained at various ratios (0% to 100%) with respect to the wild-type plasmid in Example 2, showing the sensitivity of WT probe 2 for the mutant plasmid HS38QK539L(sub2). The ordinate indicates the amount of change in the fluorescence intensity per unit time (dF/dt), and the abscissa indicates the temperature (°C).

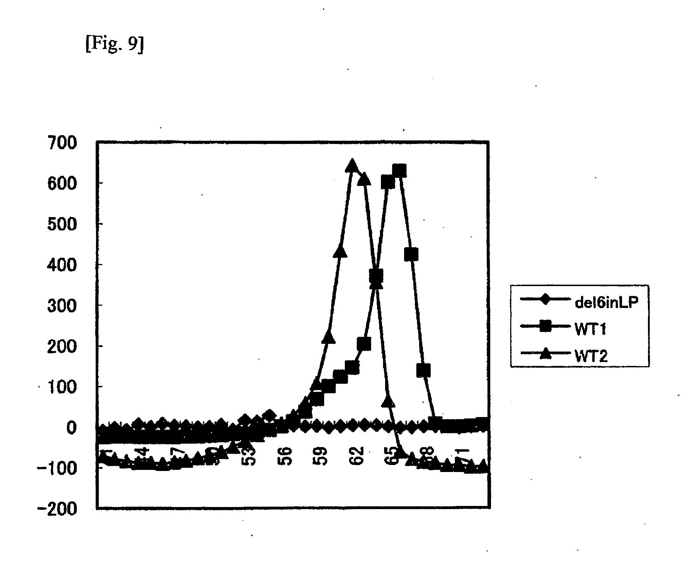

Fig. 9 shows changes in the amount of change in the fluorescence intensity per unit time (d the amount of increase in the fluorescence intensity/t) in Tm analysis for whole blood in Example 3. The ordinate indicates the amount of change in the fluorescence intensity per unit time (d the amount of increase in the fluorescence intensity/t), and the abscissa indicates the temperature (°C).

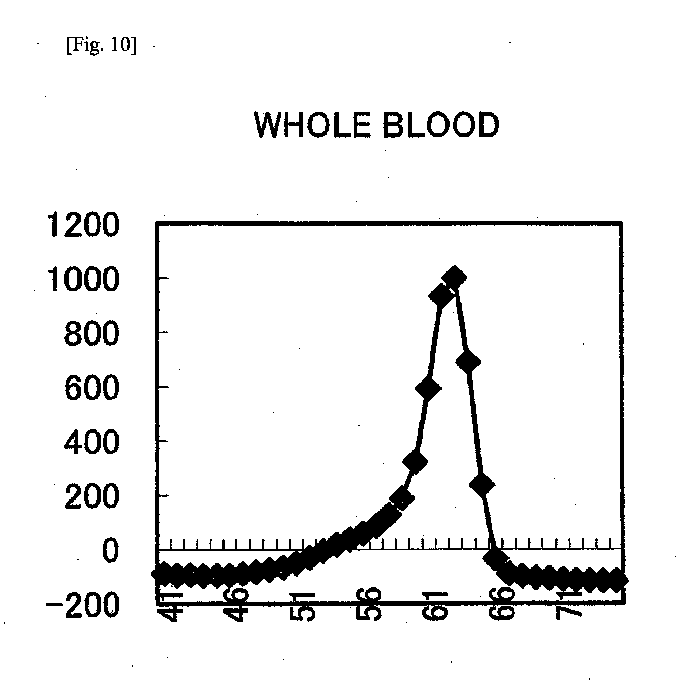

Fig. 10 shows changes in the amount of change in the fluorescence intensity per unit time (d the amount of increase in the fluorescence intensity/t) in Tm analysis for whole blood in Example 4. The ordinate indicates the amount of change in the fluorescence intensity per unit time (d the amount of increase in the fluorescence intensity/t), and the abscissa indicates the temperature (°C).

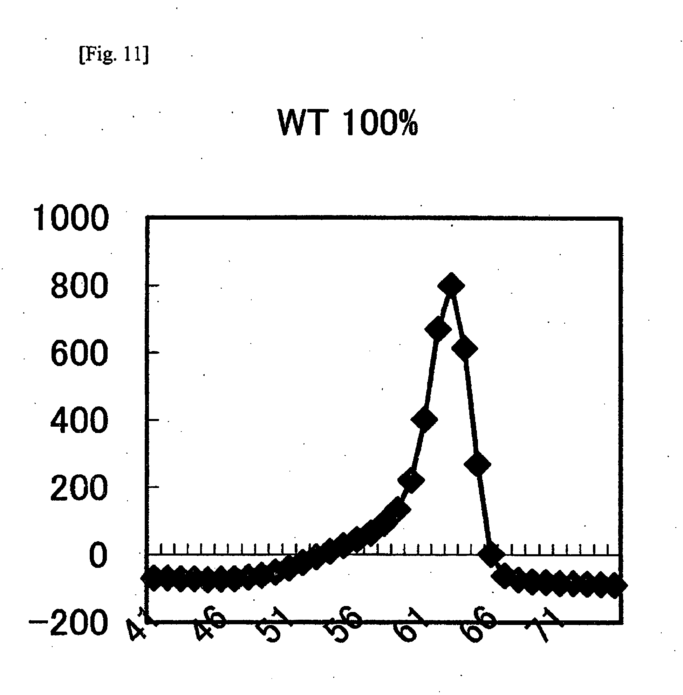

Fig. 11 shows changes in the amount of change in the fluorescence intensity per unit time (d the amount of increase in the fluorescence intensity/t) in Tm analysis for WT 100% in Example 4. The ordinate indicates the amount of change in the fluorescence intensity per unit time (d the amount of increase in the fluorescence intensity/t), and the abscissa indicates the temperature (°C).

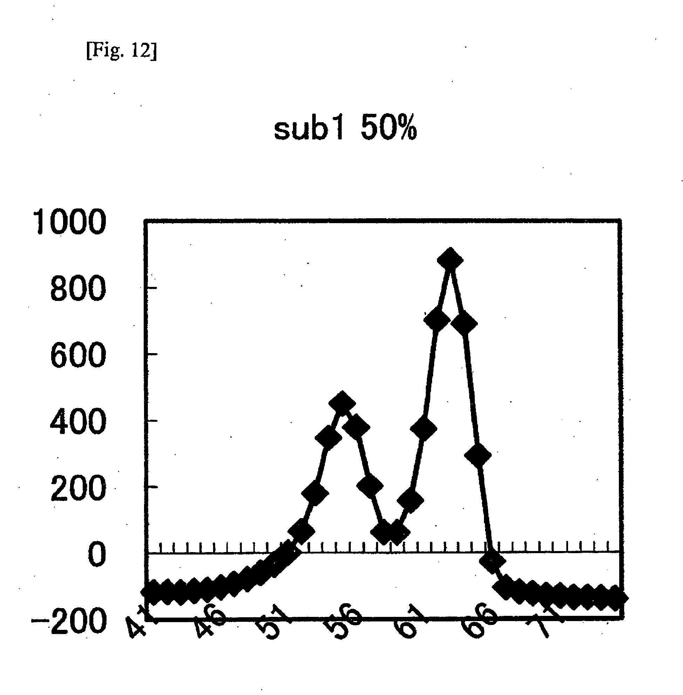

Fig. 12 shows changes in the amount of change in the fluorescence intensity per unit time (d the amount of increase in the fluorescence intensity/t) in Tm analysis for sub1 50% in Example 4. The ordinate indicates the amount of change in the fluorescence intensity per unit time (d the amount of increase in the fluorescence intensity/t), and the abscissa indicates the temperature (°C).

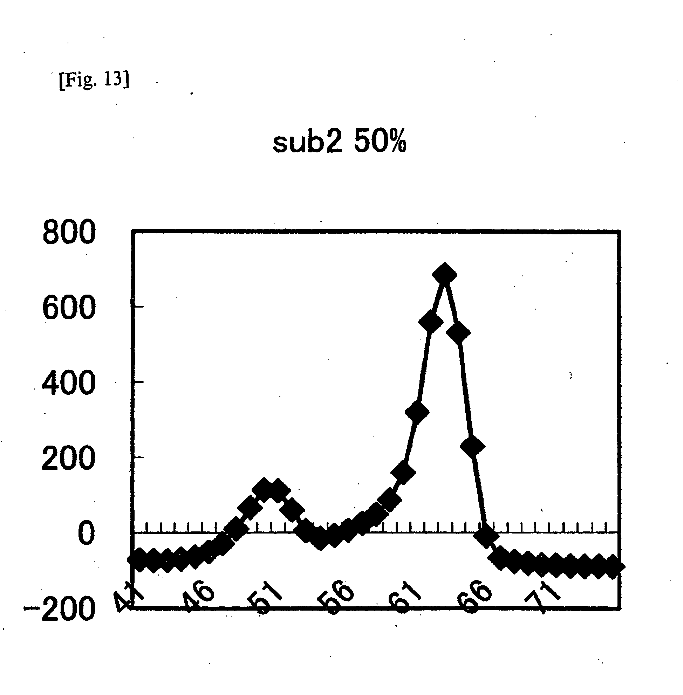

Fig. 13 shows changes in the amount of change in the fluorescence intensity per unit time (d the amount of increase in the fluorescence intensity/t) in Tm analysis for sub2 50% in Example 4. The ordinate indicates the amount of change in the fluorescence intensity per unit time (d the amount of increase in the fluorescence intensity/t), and the abscissa indicates the temperature (°C).

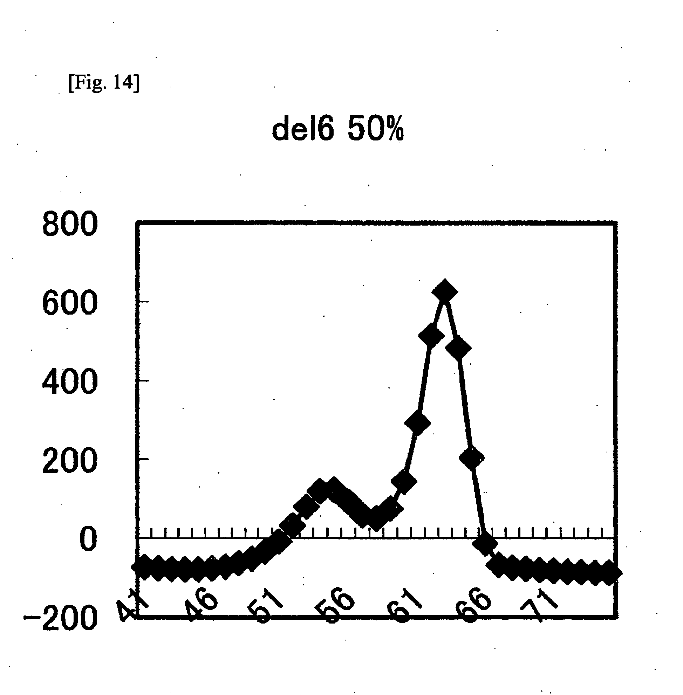

Fig. 14 shows changes in the amount of change in the fluorescence intensity per unit time (d the amount of increase in the fluorescence intensity/t) in Tm analysis for del6 50% in Example 4. The ordinate indicates the amount of change in the fluorescence intensity per unit time (d the amount of increase in the fluorescence intensity/t), and the abscissa indicates the temperature (°C).

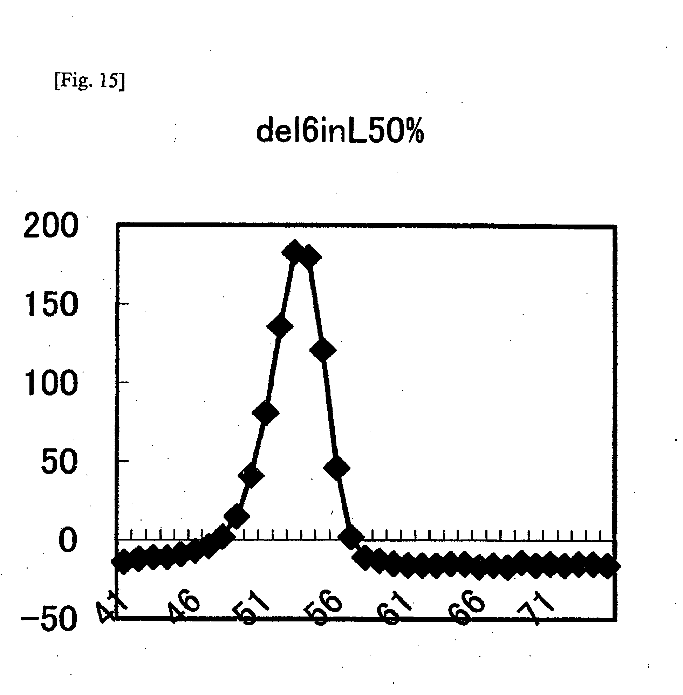

Fig. 15 shows changes in the amount of change in the fluorescence intensity per unit time (d the amount of increase in the fluorescence intensity/t) in Tm analysis for de16inL 50% in Example 5: The ordinate indicates the amount of change in the fluorescence intensity per unit time (d the amount of increase in the fluorescence intensity/t), and the abscissa indicates the temperature (°C).

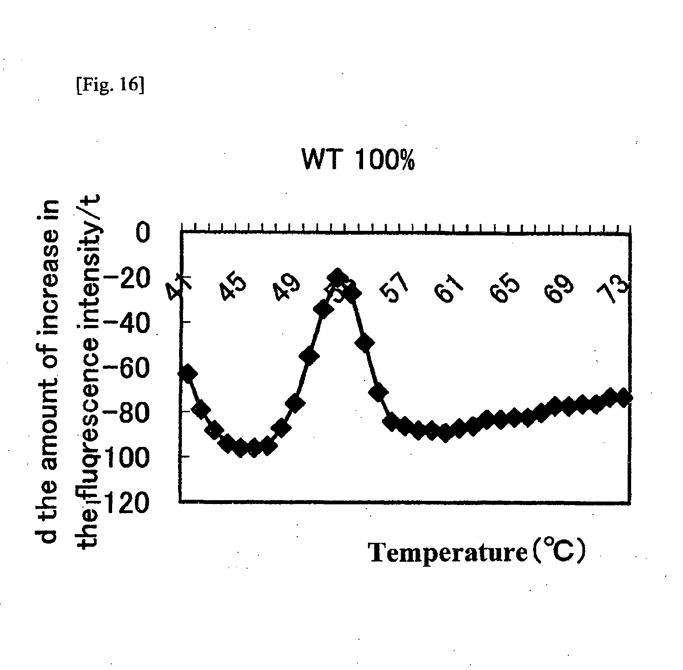

Fig. 16 shows changes in the amount of change in the fluorescence intensity per unit time (d the amount of increase in the fluorescence intensity/t) in Tm analysis for WT 100% in Comparative Example. The ordinate indicates the amount of change in the fluorescence intensity per unit time (d the amount of increase in the fluorescence intensity/t), and the abscissa indicates the temperature (°C).

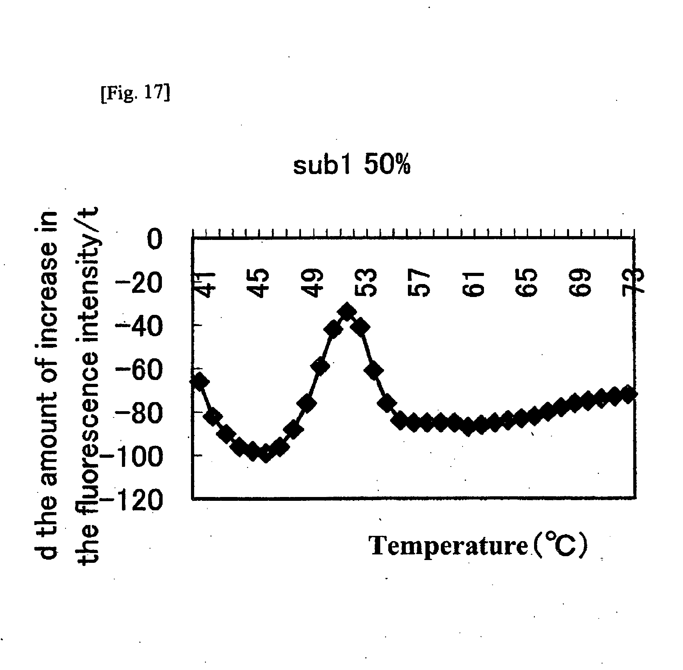

Fig. 17 shows changes in the amount of change in the fluorescence intensity per unit time (d the amount of increase in the fluorescence intensity/t) in Tm analysis for subl 50% in Comparative Example. The ordinate indicates the amount of change in the fluorescence intensity per unit time (d the amount of increase in the fluorescence intensity/t), and the abscissa indicates the temperature (°C).

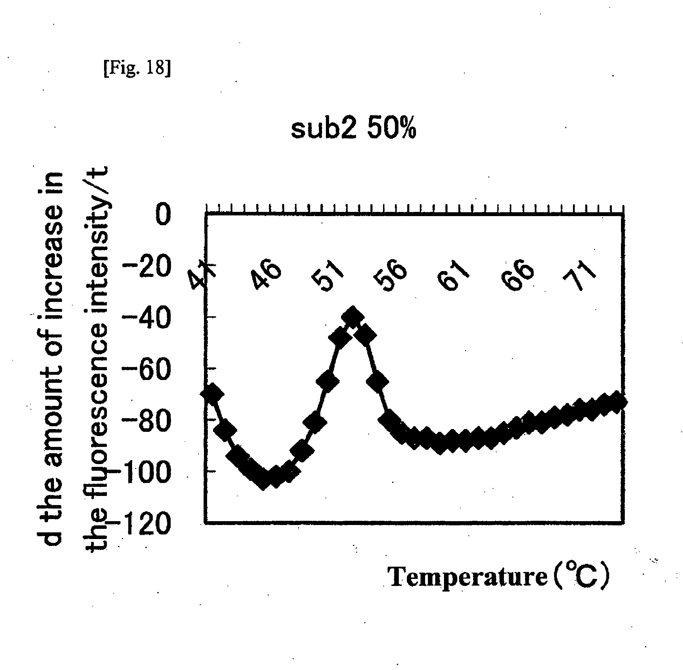

Fig. 18 shows changes in the amount of change in the fluorescence intensity per unit time (d the amount of increase in the fluorescence intensity/t) in Tm analysis for sub2 50% in Comparative Example. The ordinate indicates the amount of change in the fluorescence intensity per unit time (d the amount of increase in the fluorescence intensity/t), and the abscissa indicates the temperature (°C).

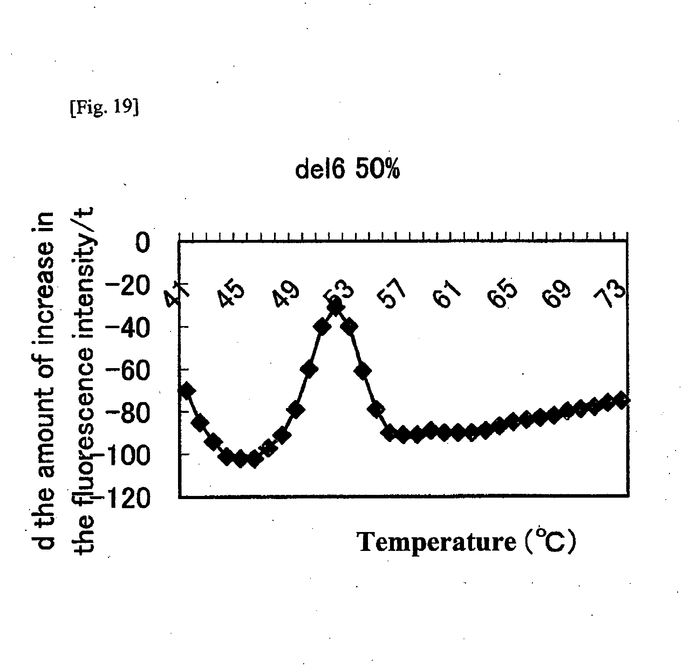

Fig. 19 shows changes in the amount of change in the fluorescence intensity per unit time (d the amount of increase in the fluorescence intensity/t) in Tm analysis for de16 50% in Comparative Example. The ordinate indicates the amount of change in the fluorescence intensity per unit time (d the amount of increase in the fluorescence intensity/t), and the abscissa indicates the temperature (°C).

DETAILED DESCRIPTION OF THE INVENTION

<1> Probe of Present Invention and Detection Method of Present Invention

A probe for detecting a polymorphism in exon 12 of the JAK2 gene, the probe comprising at least one of the oligonucleotides in (a) to (c) below:

(a) an oligonucleotide having a nucleotide sequence homologous to the sequence complementary to a nucleotide sequence having a length of 19 to 50 nucleotides containing the nucleotides 114 to 132 of SEQ ID NO: 1, wherein said oligonucleotide has the nucleotide corresponding to the nucleotide 114 labeled with a fluorescent dye at the first, second or third position from the 3' end;

(b) an oligonucleotide having a nucleotide sequence homologous to the sequence complementary to a nucleotide sequence having a length of 22 to 50 nucleotides containing the nucleotides 122 to 143 of SEQ ID NO:1, wherein said oligonucleotide has the nucleotide corresponding to the nucleotide 143 labeled with a fluorescent dye at the first, second or third position from the 5' end; and

(c) an oligonucleotide having a nucleotide sequence homologous to a nucleotide sequence having a length of 10 to 50 nucleotides containing the nucleotides 41 to 50 of SEQ ID NO:11, wherein said oligonucleotide shown in (c) has the nucleotide corresponding to the nucleotide 50 labeled with a fluorescent dye at the first, second or third position from the 3' end.

In the present specification, a complementary nucleotide sequence means that the sequence is complementary to the total length of the subject nucleotide sequence.

The probe of the present invention may be the same as the quenching probe described in JP 2001-286300 A and JP 2002-119291 A except that it has the nucleotide sequence specified in the above (a) to (c) in SEQ ID NO:1 (the sequence having the wild-type (normal-type) nucleotides in terms of the genetic variation in exon 12 of the JAK2 gene) or SEQ ID NO:11 (the sequence having the nucleotides of the F537-K539delinsL (del6inL) variant in terms of the genetic variation in exon 12 of the JAK2 gene). The length of the probe of the present invention is 10 to 50 nucleotides, preferably 15 to 35 nucleotides. More particularly, the length of the oligonucleotide (a) is preferably not less than 19 nucleotides, the length of the oligonucleotide (b) is preferably not less than 22 nucleotides, and the length of the oligonucleotide (c) is preferably not less than 10 nucleotides. Still more particularly, the length of the oligonucleotide (a) is preferably 24 to 30 nucleotides, the length of the oligonucleotide (b) is preferably 29 to 35 nucleotides, and the length of the oligonucleotide (c) is preferably 16 to 22 nucleotides. Examples of the nucleotide sequence of the quenching probe used in the present invention include those shown in SEQ ID NOs:4 to 6. The fluorescent dye may be the ones described in JP 2001-286300 A and JP 2002-119291 A, and particular examples thereof include Pacific Blue (trademark), FAM (trademark), TAMRA (trademark) and BODIPY (trademark) FL. Examples of the method for binding the fluorescent dye to the oligonucleotide include conventional methods such as the methods described in JP 2001-286300 A and JP 2002-119291 A.

The probe (a) of the present invention has a sequence having an identity of not less than 80%, more preferably not less than 90%, most preferably not less than 95% to the complementary strand of a nucleotide sequence having a length of 19 to 50 nucleotides containing the nucleotides 114 to 132. This defines the homologous sequence. That is, the probe (a) of the present invention is complementary to the nucleotide sequence having a length of 19 to 50 nucleotides containing the nucleotides 114 to 132, but does not need to be completely complementary to the nucleotide sequence and may be different for only 5, 4, 3, 2 or 1 nucleotide(s) from the complementary sequence.

The probe (b) of the present invention has a sequence having an identity of not less than 80%, more preferably not less than 90%, most preferably not less than 95% to the complementary strand of a nucleotide sequence having a length of 22 to 50 nucleotides containing the nucleotides 122 to 143. This defines the homologous sequence. That is, the probe (b) of the present invention is complementary to the nucleotide sequence having a length of 22 to 50 nucleotides containing the nucleotides 122 to 143, but does not need to be completely complementary to the nucleotide sequence and may be different for only 5, 4, 3, 2 or 1 nucleotide(s) from the complementary sequence.

The probe (c) of the present invention has a sequence having an identity of not less than 80%, more preferably not less than 90%, most preferably not less than 95% to a nucleotide sequence having a length of 10 to 50 nucleotides containing the nucleotides 41 to 50. This defines the homologous sequence. That is, the probe (c) of the present invention is homologous to the nucleotide sequence having a length of 10 to 50 nucleotides containing the nucleotides 41 to 50, but does not need to be completely identical to the nucleotide sequence and may be different for only 5, 4, 3 or 2 nucleotides.

Preferably, in the probe of the present invention, the intensity of fluorescence from the fluorescent dye decreases or increases when the probe is hybridized with the target sequence, compared with when the probe is not hybridized with the target sequence. Preferably, the probe of the present invention is a quenching probe which emits fluorescence from a fluorescent dye when the probe is not hybridized, and the fluorescence from the fluorescent dye is quenched when the probe is hybridized.

The probe of the present invention has a nucleotide labeled with a fluorescent dye at the first, second or third position from the 5' or 3' end, and the probe more preferably has the 5' or 3' end which is labeled with a fluorescent dye.

Herein, when referring to "the first to third positions from the 5' end", the 5' end itself represents the first position, and when referring to "the first to third positions from the 3' end", the 3' end itself represents the first position.

As shown in Table 8 in Examples of the present invention, a polymorphism in exon 12 of the JAK2 gene can be detected by using any one of the oligonucleotides (a) to (c), preferably by using any two of the oligonucleotides (a) to (c), more preferably by using the three oligonucleotides (a) to (c), of the present invention.

The polymorphism of exon 12 of the JAK2 gene which can be detected by the oligonucleotide of the present invention is preferably at least one mutation selected from the group consisting of E543-D544del, F537-K539delinsL, H538QK539L, K539L, N542-E543del, D544-L545del, H538DK539LI540S, H538-K539del, R541KD544N and H538-K539delinsL, most preferably at least one mutation selected from the group consisting of K539L(subl), H538QK539L(sub2), N542-E543del(de16) and F537-K539delinsL(del6inL).

Further, by using the oligonucleotide of the present invention, R541KD544N and H538-K539delinsL, which are not described in Haematologica 2009; 94(3):414-418, can also be detected. It should be noted that H538-K539delinsL is described in Haematologica 2007 Dec;92(12):1717-8 and R541KD544N has not been known yet. The sequence of R541KD544N is described below.

[Table 1]

| Name | Partial sequence around the site of mutation | SEQ ID NO | Positions |
|---|---|---|---|
| Wild type (WT) | TGGTGTTTCACAAAATCAGAAATCAAGATT | 12 | 112-141 |
| R541KD544N | TGGTGTTTCT - - - AATCAGAAATGAAGATT | 13 | |
| •The site of mutation is underlined. '-' represents a deletion.<br>•The positions for the wild type (WT) shown in the table represent the nucleotides of SEQ ID NO:1. | | | |

In cases where the oligonucleotide (a) of the present invention is used, E543-D544del, H538QK539L, K539L, N542-E543del, D544-L545del, H538DK539LI5405, H538-K539delinsL, R541KD544N and H538-K539del can be detected.

In cases where the oligonucleotide (b) of the present invention is used, H538QK539L, K539L, H538DK539LI5405, H538-K539delinsL, R541KD544N and H538-K539del can be detected.

In cases where the oligonucleotide (c) of the present invention is used, F537-K539delinsL can be detected.

By using the oligonucleotides (a) to (c) of the present invention, presence/absence of a mutation in exon 12 of the JAK2 gene can be detected, and chronic myeloproliferative disorders and polycythemia vera can be diagnosed together with diagnostic items such as the increase of hemoglobin value and erythrocyte amount.

The detection method of the present invention is a method for detecting a mutation in a nucleic acid comprising a polymorphic site in exon 12 of the JAK2 gene, comprising the step of carrying out melting curve analysis using a nucleic acid probe labeled with a fluorescent dye for measuring fluorescence from the fluorescent dye, and detecting the mutation based on the result of the melting curve analysis, wherein the nucleic acid probe is the probe of the present invention.

The detection method of the present invention can be carried out by amplifying a region containing a site of polymorphism in exon 12 of the JAK2 gene, by using a probe of the present invention, and further, by conventional nucleic acid amplification and by the method of melting curve analysis (Tm analysis).

The detection method of the present invention uses the probe of the present invention and preferably comprises the following steps:

(I) a step of adding a probe of the present invention to a sample containing DNA and allowing the probe to hybridize with the DNA;
(II) a step of dissociating the hybridized complex between the DNA and the probe by changing the temperature, and measuring fluctuation of the signal due to the dissociation of the hybridized complex;
(III) a step of determining the Tm value by analyzing the fluctuation of the signal; and
(IV) a step of determining presence/absence of a polymorphism of interest or the abundance ratios of nucleotide sequences comprising the polymorphism, based on the Tm value.

In addition to use of the probe of the present invention, the detection method of the present invention can be carried out by conventional nucleic acid amplification and the method of melting curve analysis (Tm analysis). Further, the detection method of the present invention may also comprise amplifying the nucleic acid before the above Step (I) or at the same time with the above Step (I).

The method of amplifying the nucleic acid preferably uses a polymerase, and examples thereof include PCR, ICAN and LAMP. When the amplification is carried out by a method using a polymerase, the amplification is preferably carried out in the presence of the probe of the present invention. Those skilled in the art can easily adjust the reaction conditions or the like of the amplification depending on the probe to be used. By this, the detection can be carried out just by analyzing Tm of the probe after the amplification of the nucleic acid, so that the amplification product does not need to be handled after the reaction. Therefore, there is no risk of contamination by the amplified product. Further, since the detection can be carried out with the same apparatus as the one necessary for the amplification, it is not necessary even to transfer the container. Therefore, automation can also be easily done.

The determination of the Tm value in Step (III) includes not only determination of the temperature of Tm but also determination of the height of the peak of Tm. By the height of the peak, the abundance ratios of the nucleotide sequences comprising a polymorphism can be determined. For more quantitative determination of the abundance ratios of the nucleotide sequences comprising a polymorphism, it is preferred to prepare a calibration curve as shown below and determine the abundance ratios based on the prepared calibration curve.

The method of quantitative determination of the abundance ratios of the nucleotide sequences comprising a polymorphism is shown below by way of an example of determination of the abundance ratios of the wild type and a particular variant. However, this is only an example, and the method of determination of the abundance ratios of the nucleotide sequences comprising a polymorphism is not restricted thereto.

First, multiple nucleic acid mixtures in which two types of nucleic acids, that is, the wild-type nucleic acid Wt and a variant nucleic acid Mt, are contained at various abundance ratios are prepared, and a melting curve is obtained for each of the multiple nucleic acid mixtures using a melting curve analysis device or the like.

Fig. 1 (A) shows a melting curve represented as the relationship between the temperature and the fluorescence intensity for a certain nucleic acid mixture, and Fig. 1 (B) shows a melting curve represented as the relationship between the temperature and the differential value of the fluorescence intensity (also referred to as a differential melting curve). By detecting a peak from this differential melting curve, $Tm_W$, which is the melting temperature of the nucleic acid Wt, and $Tm_M$, which is the melting temperature of the nucleic acid Mt, are detected, and each of the temperature ranges including $Tm_W$ and $Tm_M$ are set. For example, as $\Delta T_W$, which is the temperature range including $Tm_W$, the temperature range whose lower limit is the temperature at which the differential value of the fluorescence intensity is minimum between $Tm_W$ and $Tm_M$ and whose upper limit is the temperature corresponding to the skirt of the peak of the fluorescence intensity can be set. Further, for example, as $\Delta T_M$, which is the temperature range including $Tm_M$, the temperature range whose upper limit is the temperature at which the differential value of the fluorescence intensity is minimum between $Tm_W$ and $Tm_M$ and whose lower limit is the temperature corresponding to the skirt of the peak of the fluorescence intensity can be set. The temperature range $\Delta T_W$ and the temperature range $\Delta T_M$ may be set such that these have either the same width (e.g., 10°C) or different widths (e.g., a temperature range $\Delta T_W$ of 10°C and a temperature range $\Delta T_M$ of 7°C). Further, the temperature range $\Delta T_W$ and the temperature range $\Delta T_M$ may be set such that each of these has a width ranging from X°C higher than the melting temperature Tm to X°C lower than the melting temperature Tm (e.g., X°C is not more than 15°C, preferably not more than 10°C).

Subsequently, for each of the temperature range $\Delta T_W$ and the temperature range $\Delta T_M$, the area surrounded by the line passing through the point corresponding to the lower limit and the point corresponding to the upper limit of the temperature range of the differential melting curve, and the differential melting curve (shaded portion in Fig. 1(B)) is calculated. More particularly, for example, the area can be calculated as follows. By defining the differential value of the fluorescence intensity at temperature T as f(T) and the base value at temperature T as B(T), the area is calculated by the Equation (1) below.

$$\text{Area } S = \{f(T_{s+1}) - B(T_{s+1})\} + \{f(T_{s+2}) - B(T_{s+2})\} + \ldots + \{f(T_{e-1}) - B(T_{e-1})\} \ldots (1)$$

In the equation, $T_s$ represents the lower limit value of each temperature range, and $T_e$ represents the upper limit value. The base value B(T) at each temperature T is a value calculated by the Equation (2) below and represents the background level contained in the detection signal of the fluorescence intensity. By subtracting this base value from the differential value of the fluorescence intensity, the effect of the background contained in the detection signal of the fluorescence intensity is removed.

$$B(T) = a \times (T - T_s) + f(T_s) \quad \cdots (2)$$

In this equation, $a = \{f(T_e) - f(T_s)\}/(T_e - T_s)$.

According to the above Equation (1) and Equation (2), with respect to the respective mixtures, the area $S_W$ in the temperature range $\Delta T_W$ and the area $S_M$ in the temperature range $\Delta T_W$ are calculated, to prepare a calibration curve representing the relationship between the area ratio and the abundance ratio of the respective mixtures. Fig. 2 shows an example of the calibration curve prepared by taking the abundance ratio (the ratio of nucleic acid Mt with respect to the total amount of the nucleic acid mixture) along the abscissa and the area ratio ($S_M/S_W$) along the ordinate. The area ratio may also be defined as $S_W/S_M$.

By calculating the area ratio from the melting curve and the differential melting curve obtained using an actual sample and preliminarily preparing a calibration curve as described above, the abundance ratios of the nucleotide sequences comprising a polymorphism contained in the actual sample can be determined based on the prepared calibration curve.

In the present invention, the DNA in the sample may be either a single-stranded DNA or a double-stranded DNA. In cases where the DNA is a double-stranded DNA, for example, a step of dissociating the double-stranded DNA in the sample by heating is preferably included before the hybridization step. By dissociating the double-stranded DNA into single-stranded DNAs, hybridization with the detection probe is possible in the subsequent hybridization step.

In the present invention, the ratio (molar ratio) of the probe of the present invention to be added with respect to DNA in the sample is not restricted, and the ratio is preferably not more than 1, more preferably not more than 0.3 with respect to DNA in the sample to secure a sufficient detection signal. In this case, for example, the DNA in the sample may be either the total of the DNA comprising a polymorphism to be detected and DNA which does not comprise the polymorphism to be detected, or the total of the amplification product containing the sequence comprising a polymorphism to be detected and amplification products containing sequences which do not comprise the polymorphism to be detected. Although the ratio of the DNA to be detected in the DNA in the sample is usually not known, the ratio (molar ratio) of the probe to be added with respect to the DNA to be detected (the amplification product containing the sequence to be detected) is preferably not more than 100, more preferably not more than 50, still more preferably not more than 30 as a result. The lower limit of the ratio is not restricted, and the ratio is, for example, not less than 0.001, preferably not less than 0.01, more preferably not less than 0.2.

The ratio of the probe of the present invention to be added with respect to the DNA may be, for example, either a molar ratio with respect to the double-stranded DNA or a molar ratio with respect to the single-stranded DNA.

The Tm value will now be described. Heating a solution containing double-stranded DNA causes an increase in the absorbance at 260 nm. This is caused because the hydrogen bond between the both strands of the double-stranded DNA is unraveled by the heat and the double-stranded DNA is dissociated into single-stranded DNAs (melting of DNA). When all the double-stranded DNAs are dissociated into single-stranded DNAs, the absorbance becomes about 1.5 times as large as that observed when the heating was initiated (absorbance for only double-stranded DNA), and by this, completion of the melting can be judged. Based on this phenomenon, the melting temperature Tm is defined as the temperature at which the increase in absorbance reaches 50% of the total increase in absorbance.

Measurement of the signal fluctuation due to the temperature change for determination of the Tm value can be carried out by measuring the absorbance at 260 nm based on the above-mentioned principle, but the measurement is preferably carried out based on a signal from the label added to the probe of the present invention, which signal fluctuates depending on the state of hybrid formation between the DNA and the probe. Therefore, as the probe of the present invention, the above-mentioned labeled probe is used. Examples of the labeled probe include a fluorescently labeled oligonucleotide probe wherein the probe emits fluorescence when it is not hybridized with the target sequence and the fluorescence intensity decreases (the fluorescence is quenched) when the probe is hybridized with the target sequence, and a fluorescently labeled oligonucleotide probe wherein the probe emits fluorescence when it is not hybridized with the target sequence and the fluorescence intensity increases when the probe is hybridized with the target sequence. In the case of the former probe, the probe shows no signal or a weak signal when it is forming a hybrid (double-stranded DNA) with the sequence to be detected, while the probe shows a signal or the signal increases when the probe is released by heating. In the case of the latter probe, the probe shows a signal by forming a hybrid (double-stranded DNA) with the sequence to be detected, while the signal decreases (disappears) when the probe is released by heating. Therefore, by detecting the change in the signal due to the label under conditions specific to the signal (absorbance and the like), the progress of melting and the determination of Tm value can be carried out similarly to the case of the measurement of the absorbance at 260 nm. The probe according to the invention is a fluorescent dye-labeled probe.

The method will now be further described by way of an example using PCR. The primer pair used in the PCR may be designed in the same manner as in the method of designing a primer pair in a conventional PCR except that the primer pair is designed such that the region with which the probe of the present invention can be hybridized is amplified. The length and Tm of each primer are usually 12-mer to 40-mer and 40 to 70°C, preferably 16-mer to 30-mer and 55 to 60°C. The lengths of the respective primers of the primer pair do not need to be the same, but the Tm values of the both primers are preferably almost the same (the difference is usually within 2°C). Here, the Tm value is calculated by the Nearest Neighbor method. Examples of the primer pair include the one composed of the primers having the nucleotide sequences shown in SEQ ID NOs:2 and 3.

The PCR is preferably carried out in the presence of the probe of the present invention to be used in the present invention. By this, Tm analysis can be carried out after the amplification reaction without handling the amplified product. According to probes to be used, those skilled in the art can easily adjust the Tm values of the primers and the reaction conditions for the PCR.

A representative temperature cycle is as follows, which temperature cycle is usually repeated 25 to 50 times.

    (1) Denaturation, 90 to 98°C, 1 to 60 seconds
    (2) Annealing, 50 to 70°C, 10 to 60 seconds
    (3) Extension, 60 to 75°C, 10 to 180 seconds

In cases where annealing and extension are carried out in a single step, the conditions are 55 to 70°C and 10 to 180 seconds, for example.

The Tm analysis can be carried out in the same manner as in a conventional method except that fluorescence from the fluorescent dye of the probe of the present invention is measured. The measurement of fluorescence can be carried out by measuring light having the emission wavelength using an excitation light having a wavelength dependent on the fluorescent dye. The heating rate in the Tm analysis is usually 0.1 to 1°C/second. The composition of the reaction liquid used for carrying out the Tm analysis is not restricted as long as the probe can be hybridized with a nucleic acid having the complementary sequence of the nucleotide sequence of the probe, and usually, the concentration of monovalent cations is 1.5 to 5 mM, and pH is 7 to 9. Since the reaction liquid for an amplification method using a DNA polymerase, such as PCR, usually satisfies these conditions, the reaction liquid after the amplification can be used as it is for the Tm analysis.

Detection of the mutation in exon 12 of the JAK2 gene based on the result of the Tm analysis can be carried out according to a conventional method. The detection in the present invention includes detection of presence/absence of a mutation. Since detection of presence/absence of a mutation is possible by the probe and the method of the present invention, diagnosis of chronic myeloproliferative disorders and polycythemia vera is also included in the present invention.

<2> Kit of Present Invention

The kit of the present invention is a kit for use in the detection method of the present invention. This kit contains the probe of the present invention for detecting a polymorphism. The kit of the present invention can be used also for diagnosis of chronic myeloproliferative disorders and polycythemia vera.

The quenching probe is as described above for the probe of the present invention.

The detection kit of the present invention may include, in addition to the quenching probe, reagents necessary for nucleic acid amplification in the detection method of the present invention, especially primers for amplification using a DNA polymerase.

In the detection kit of the present invention, the quenching probe, the primers and the other reagents may be contained separately, or a part thereof may be contained as a mixture.

The present invention will now be described concretely by way of Examples.

EXAMPLES

Example 1 (Plasmid is used as a target with the 3 probes)

Based on the nucleotide sequence (SEQ ID NO:1 (wild type)) containing the region of a polymorphism in exon 12 of the JAK2 gene, the primers shown in Table 2 were designed such that the region of the polymorphism can be amplified. In Table 2, the positions represent the nucleotides of SEQ ID NO: 1.

[Table 2]

| Primers | | | | |
|---------|---------------------------------------|-----|----------|----------|
| Name | Sequence (5'-3') | Mer | Positions | SEQ ID NO |
| F | ctgatgtaccaacctcaccaacattacagag | 31 | 61-91 | 2 |
| R | aaggacaaaaaagacagtaatgagtatctaatgac | 35 | 188-154 | 3 |

Subsequently, probes having C in their ends were designed as shown in Table 3. In Table 3, the positions for the WT probes 1 and 2 represent the nucleotides of SEQ ID NO:1. The positions for del6inL probe represent the nucleotides of SEQ ID NO:11.

P in the 3' end means that the end is phosphorylated. Labeling with Pacific Blue, BODIPY FL or TAMRA was carried out according to a conventional method.

[Table 3]

| Name | Sequence (5'-3') | Mer | Positions | SEQ ID NO |
|-------------|------------------------------------------------|-----|----------|----------|
| del6inL probe | cttcatttctgattaacac-(Pacific Blue) | 19 | 32-50 | 4 |
| WT probe 1 | (BODIPY FL)-caaatcttcatttctgattttgtgaaacacca-P | 32 | 143-112 | 5 |
| WT probe 2 | atcttcatttctgattttgtgaaacac-(TAMRA) | 27 | 140-114 | 6 |

Using a fully automatic SNPs testing device (trade name: i-densy (trademark), manufactured by ARKRAY, Inc.), 50 μL of a mixture containing $10^4$ copies of the samples shown in Table 5 prepared by adding the respective components of the PCR reaction liquid shown in Table 4 to the following concentrations was subjected to PCR and Tm analysis. The conditions for the PCR and the Tm analysis were as described below. The excitation wavelength and the detection wavelength in the Tm analysis were 365 to 415 nm and 445 to 480 nm, respectively (Pacific Blue); 420 to 485 nm and 520 to 555 nm, respectively (BODIPY FL); or 520 to 555 nm and 585 to 700 nm, respectively (TAMRA).

[Table 4]

In 50 μL of the reagent:

| | |
|---|---|
| 1× | Reaction buffer |
| 1.25 U | Taq polymerase |
| 1.5 mmol/L | MgCl$_2$ |
| 0.2 mmol/L | dNTP |
| 1 μmol/L | F primer |
| 0.5 μmol/L | R primer |
| 0.2 μmol/L | WT probe 1 |
| 0.2 μmol/L | WT probe 2 |
| 0.2 μmol/L | del6inL probe |

[Table 5]

| Sample | Mixing ratios of the respective plasmids | | | | |
|---|---|---|---|---|---|
| Sample name | Wt | K539L (sub1) | H538QK539L (sub2) | N542-E543del (del6) | F537-K539delinsL (del6inL) |
| Wt 100% | 100% | 0% | 0% | 0% | 0% |
| sub 150% | 50% | 50% | 0% | 0% | 0% |
| sub2 50% | 50% | 0% | 50% | 0% | 0% |
| del6 50% | 50% | 0% | 0% | 50% | 0% |
| del6inL 50% | 50% | 0% | 0% | 0% | 50% |

[Table 6]

| Name | Partial sequence around the site of mutation | SEQ ID NO | Positions |
|---|---|---|---|
| Wild type (WT) | atgagtatctaatgacttacaaatatcaaatc**ttcatt**tctgatt**ttgtgaaa**caccat | 7 | 169-111 |
| K539L(sub1) | atgagtatctaatgacttacaaatatcaaatc**ttcatt**tctgatt**aagtgaaa**caccat | 8 | |
| H538QK539L(sub2) | atgagtatctaatgacttacaaatatcaaatc**ttcatt**tctgatt**aattgaaa**caccat | 9 | |
| N542-E543 del(del6) | atgagtatctaatgacttacaaatatcaaatc------tctgatt**ttgtgaaa**caccat | 10 | |
| F537-K539delinsL (del6inL) | atgagtatctaatgacttacaaatatcaaatc**ttcatt**tctgatt**aa**-caccat | 11 | |
| •All the sequences are shown as reverse sequences. •Mutated sites are underlined. '-' represents a deletion. the table, the positions for the wild type (WT) are the nucleotides of SEQ ID NO:1. | | | |

[Table 7]

**Conditions for PCR and Tm analysis**

95°C, 60sec

↓

(95°C, 1 sec.; 60°C, 15 sec.)×50

↓

(continued)

**Conditions for PCR and Tm analysis**
95°C, 1 sec.
↓
40°C, 60 sec.
↓
Tm (40°C→75°C, 1°C/3 sec.)

As a result of PCR and Tm analysis using the 3 probes in Table 3, in terms of the Wt 100% sample, the peak for TAMRA (WT probe 2) was observed at about 63°C, the peak for BODIPY FL (WT probe 1) was observed at about 66°C, and the peak for Pacific Blue (de16inL probe) was not observed (Fig. 3).

In terms of the sub1 50% sample, in addition to the peaks observed for the WT plasmid, the peak for TAMRA (WT probe 2) was observed at about 55°C, the peak for BODIPY FL (WT probe 1) was observed at about 60°C, and the peak for Pacific Blue (del6inL probe) was not observed (Fig. 4).

In terms of the sub2 50% sample, in addition to the peaks observed for the WT plasmid, the peak for TAMRA (WT probe 2) was observed at about 50°C, the peak for BODIPY FL (WT probe 1) was observed at about 57°C, and the peak for Pacific Blue (del6inL probe) was not observed (Fig: 5).

In terms of the del6 50% sample, in addition to the peaks observed for the WT plasmid, the peak for TAMRA (WT probe 2) was observed at about 54°C, but no other peak for BODIPY FL (WT probe 1) was observed, and the peak for Pacific Blue (del6inL probe) was also not observed (Fig. 6).

In terms of the del6inL 50% sample, in addition to the peaks observed for the WT plasmid, another peak for BODIPY FL (WT probe 1) was observed at about 52°C, and the peak for Pacific Blue (del6inL probe) was observed at about 53°C, but no other peak for TAMRA (WT probe 2) was observed (Fig. 7).

These results are shown in Table 8 below.

## [Table 8]

o Summary of Tm Values

| Mutation<br>Wavelength | WT | K539L (sub1) | H538QK539L (sub2) | N542-E543del (del6) | F537-K539 delinsL(del6inL) |
|---|---|---|---|---|---|
| TAMRA (WT probe 2) | About 63°C | 55°C | 50°C | 54°C | No peak |
| BODIPY FL (WT probe 1) | 66°C | 60°C | 57°C | No peak | 52°C |
| Pacific Blue (del6inL probe) | No peak | No peak | No peak | No peak | 53°C |

When the 3 probes of SEQ ID NOs:4 to 6 were used at the same time, changes in the fluorescence intensity which could be analyzed by the Tm analysis were observed for the mutations sub1, sub2, del6 and del6inL, and different patterns of changes were observed for these mutations. That is, a unique change in the pattern of the amount of change in the fluorescence intensity exists for each mutation.

Example 2 (Detection Sensitivity)

The results of Tm analysis carried out by changing the ratio (100% to 0%) of the mutant plasmid H538QK539L(sub2) with respect to the wild-type plasmid are shown in Fig. 8. In the case of H538QK539L(sub2) 100%, an evident high peak was observed at about 50°C, in the cases ofH538QK539L(sub2) 5 to 20% (that is, wt 95 to 80%), a peak was observed at about 63°C together with a peak at about 50°C, and in the cases of H538QK539L(sub2) 1 and 0% (that is, wt 99 and 100%), an evident high peak was observed at about 63°C. From these results, it can be seen that this quantification method is highly sensitive.

This analysis was carried out in the same manner as in the above Example 1 except that $10^4$ copies of the sample was added to the reaction mixture only in the case of H538QK539L(sub2) 1% and $10^3$ copies of the sample was added in the other cases.

Example 3 (Whole blood is used as a target with the 3 probes)

Whole blood from a healthy subject was used as the sample, and pretreatment PCR for the whole blood and Tm analysis were carried out using a fully automatic SNPs testing device (trade name: i-densy (trademark), manufactured by ARKRAY, Inc.) and i-densy Pack UNIVERSAL (trademark, manufactured by ARKRAY, Inc.). The primer/probe solution aliquoted to i-densy Pack UNIVERSAL (trademark, manufactured by ARKRAY, Inc.) was as shown in Table 9. The conditions for the PCR and the Tm analysis were as described above.

[Table 9]

| In the primer/probe solution: | |
| --- | --- |
| 27 mmol/L | Tris-HCl (pH 8.6) |
| 5 $\mu$mol/L | F primer |
| 2.5 $\mu$mol/L | R primer |
| 1 $\mu$mol/L | WT probe 1 |
| 1 $\mu$mol/L | WT probe 2 |
| 1 $\mu$mol/L | del6inL probe |

As a result of PCR and Tm analysis using the 3 probes in Table 3, in terms of the whole blood, the peak for TAMRA (WT probe 2) was observed at about 63°C, the peak for BODIPY FL (WT probe 1) was observed at about 66°C, and the peak for Pacific Blue (del6inL probe) was not observed, as in the case of the WT plasmid (Fig. 9).

Therefore, since the wild type shows similar patterns of change for both the plasmid and the whole blood, it can be understood that the probe of the present invention can be applied to detection of a mutation using not only a plasmid but also whole blood.

Example 4 (Whole blood or Plasmid is used as a target with WT probe 2 alone)

Using a fully automatic SNPs testing device (trade name: i-densy (trademark), manufactured by ARKRAY, Inc.), 50 $\mu$L of a mixture containing $10^4$ copies of the samples shown in Table 5 (WT 100%, sub 50%, sub2 50%, or de16 50%) prepared by adding the respective components of the PCR reaction liquid shown in Table 10 to the following concentrations was subjected to PCR and Tm analysis. The conditions for the PCR and the Tm analysis were as described above.

As for whole blood, the experiment was performed in the same way as in Example 3, except that PCR reaction liquid shown in Table 10 was used.

[Table 10]

| In 50 $\mu$L of the reagent: | |
| --- | --- |
| 1 × | Reaction buffer |
| 1.25 U | Taq polymerase |
| 1.5 mmol/L | $MgCl_2$ |
| 0.2 mmol/L | dNTP |
| 1 $\mu$mol/L | F primer |
| 0.5 $\mu$mol/L | R primer |
| 0.2 $\mu$mol/L | WT probe 2 |

As a result of PCR and Tm analysis using the 3 probes in Table 3, in terms of the Whole Blood, as in Example 3, peak for TAMRA (WT probe 2) was observed at about 63°C (Fig. 10).

In terms of the Wt 100% sample, as in Example 1, the peak for TAMRA (WT probe 2) was observed at about 63°C (Fig. 11).

In terms of the sub1 50% sample, as in Example 1, in addition to the peaks observed for the WT plasmid, the peak for TAMRA (WT probe 2) was observed at about 55°C (Fig. 12).

In terms of the sub2 50% sample, as in Example 1, in addition to the peaks observed for the WT plasmid, the peak for TAMRA (WT probe 2) was observed at about 50°C (Fig. 13).

In terms of the del6 50% sample, as in Example 1, in addition to the peaks observed for the WT plasmid, the peak for TAMRA (WT probe 2) was observed at about 54°C (Fig. 14).

Therefore, since similar results as in Examples 1 and 3 were obtained when only one probe (WT probe 2) was used, it can be understood that using only one of the probes of the present invention allows detection of a mutation.

Example 5 (Plasmid is used as a target with del6inL probe alone)

Using a fully automatic SNPs testing device (trade name: i-densy (trademark), manufactured by ARKRAY, Inc.), 50 μL of a mixture containing $10^4$ copies of the samples shown in Table 5 (de16inL 50%) prepared by adding the respective components of the PCR reaction liquid shown in Table 11 to the following concentrations was subjected to PCR and Tm analysis. The conditions for the PCR and the Tm analysis were as described above.

[Table 11]

In 50 μL of the reagent:

| | |
|---|---|
| 1 × | Reaction buffer |
| 1.25 U | Taq polymerase |
| 1.5 mmol/L | MgCl$_2$ |
| 0.2 mmol/L | dNTP |
| 1 μmol/L | F primer |
| 0.5 μmol/L | R primer |
| 0.2 μmol/L | Del6inL probe |

As a result of PCR and Tm analysis using del6inL probe in Table 3, in terms of the del6inL 50% sample, the peak for Pacific Blue (del6inL probe) was observed at about 53°C (Fig. 15).
Therefore, since the similar results as Example 1 were obtained when only one probe was used, it can be understood that using only one of the probes of the present invention allows detection of a mutation.

Comparative Example (Plasmid is used as a target with WT probe 3 alone)

Using a fully automatic SNPs testing device (trade name: i-densy (trademark), manufactured by ARKRAY, Inc.), 50 μL of a mixture containing $10^4$ copies of the samples shown in Table 5 (WT 100%, sub1 50%, sub2 50%, or del6 50%) prepared by adding the respective components of the PCR reaction liquid shown in Table 12 to the following concentrations was subjected to PCR and Tm analysis. The conditions for the PCR and the Tm analysis were as described above.
Namely, this experiment was performed in the same way as in Example 4, except that WT probe 3 was used.

[Table 12]

In 50 μL of the reagent:

| | |
|---|---|
| 1 × | Reaction buffer |
| 1.25 U | Taq polymerase |
| 1.5 mmol/L | MgCl$_2$ |
| 0.2 mmol/L | dNTP |
| 1 μmol/L | F primer |
| 0.5 μmol/L | R primer |
| 0.2 μmol/L | WT probe 3 |

[Table 13]

| Name | sequence(5'-3') | mer | The position in SEQ ID NO:1 | SEQ ID NO |
|---|---|---|---|---|
| WT probe 3 | (TAMRA)-cagaaatgaagatttgat-P | 18 | 128-145 | 14 |

As a result of PCR and Tm analysis using WT probe 3 alone in Table 13, in terms of the Wt 100%, sub1 50%, sub2 50%, and del6 50% samples, similar waveforms were observed (Figs. 16-19). That is, none of the mutations were detected.
Therefore, it can be understood that any probe labeled with a fluorescent dye at 5' or 3' end does not necessarily detect a mutation, and thus the nucleotide 143 or 114 of cytosine should be labeled with a fluorescent dye, as in the probe of SEQ ID NO: 5 or 6.

INDUSTRIAL APPLICABILITY

By using the probe of the present invention, the presence/absence of a mutation in exon 12 of the JAK2 gene can be detected.

## Claims

1.  A probe for detecting a polymorphism in exon 12 of the JAK2 gene, said probe comprising at least one of the oligonucleotides in (a) to (c) below:

    (a) an oligonucleotide having a nucleotide sequence homologous to the sequence complementary to a nucleotide sequence having a length of 19 to 50 nucleotides containing the nucleotides 114 to 132 of SEQ ID NO:1, wherein said oligonucleotide has the nucleotide corresponding to nucleotide 114 labeled with a fluorescent dye at the first, second or third position from the 3' end;
    (b) an oligonucleotide having a nucleotide sequence homologous to the sequence complementary to a nucleotide sequence having a length of 22 to 50 nucleotides containing the nucleotides 122 to 143 of SEQ ID NO:1, wherein said oligonucleotide has the nucleotide corresponding to nucleotide 143 labeled with a fluorescent dye at the first, second or third position from the 5' end; and
    (c) an oligonucleotide having a nucleotide sequence homologous to a nucleotide sequence having a length of 10 to 50 nucleotides containing the nucleotides 41 to 50 of SEQ ID NO:11, wherein said oligonucleotide has the nucleotide corresponding to nucleotide 50 labeled with a fluorescent dye at the first, second or third position from the 3' end

2. The probe according to claim 1, wherein
said oligonucleotide shown in (a) has the nucleotide corresponding to nucleotide 114 labeled with a fluorescent dye at the 3' end;
said oligonucleotide shown in (b) has the nucleotide corresponding to nucleotide 143 at the 5' end; and/or
said oligonucleotide shown in (c) has the nucleotide corresponding to nucleotide 50 at the 3' end.

3. The probe according to claim 1 or 2, wherein the fluorescence intensity decreases or increases when said oligonucleotide is hybridized with the target sequence, compared with when said oligonucleotide is not hybridized with the target sequence.

4. The probe according to claim 3, wherein the fluorescence intensity decreases when said oligonucleotide is hybridized with the target sequence.

5. The probe according to any one of claims 1 to 4, wherein
said oligonucleotide shown in (a) has a length of 24 to 30 nucleotides;
said oligonucleotide shown in (b) has a length of 29 to 35 nucleotides; and/or
said oligonucleotide shown in (c) has a length of 16 to 22 nucleotides.

6. The probe according to any one of claims 1 to 4, wherein
said oligonucleotide shown in (a) has the nucleotide sequence shown in SEQ ID NO:6;
said oligonucleotide shown in (b) has the nucleotide sequence shown in SEQ ID NO:5; and/or
said oligonucleotide shown in (c) has the nucleotide sequence shown in SEQ ID NO:4.

7. The probe according to any one of claims 1 to 6, wherein said polymorphism in exon 12 of the JAK2 gene is at least one mutation selected from the group consisting of K539L(sub1), H538QK539L(sub2), N542-E543del(de16) and F537-K539delinsL(del6inL).

8. A method for detecting a mutation in a nucleic acid comprising a polymorphic site in exon 12 of the JAK2 gene, comprising the step of carrying out melting curve analysis using a nucleic acid probe labeled with a fluorescent dye for measuring fluorescence from the fluorescent dye, and detecting the mutation based on the result of the melting curve analysis, wherein the nucleic acid probe is the probe according to any one of claims 1 to 7.

9. The method according to claim 8, further comprising the step of obtaining the nucleic acid comprising a polymorphism by amplifying the region containing the polymorphic site in the nucleic acid contained in a sample.

10. The method according to claim 9, wherein the amplification is carried out by a method using a DNA polymerase.

11. The method according to claim 10, wherein the amplification is carried out in the presence of said probe.

12. The probe of any one of claims 1 to 7 or the method according to any of claims 8 to 11 for use in the diagnosis of chronic mycloproliferative disorders or polycythemia vera.

13. A kit for detecting a polymorphism in exon 12 of the JAK2 gene, comprising the probe according to any one of claims 1 to 7.

14. The kit according to claim 13, further comprising a primer for amplifying the region containing the polymorphic site in exon 12 of the JAK2 gene by a method using a DNA polymerase.

**Patentansprüche**

1. Sonde zum Erfassen eines Polymorphismus in Exon 12 des JAK2-Gens, welche Sonde mindestens eines der Oligonukleotide in den nachstehenden Absätzen (a) bis (c) enthält:

(a) ein Oligonukleotid, welches eine Nukleotidsequenz hat, die zu der Sequenz homolog ist, welche zu einer Nukleotidsequenz mit einer Länge von 19 bis 50 Nukleotiden komplementär ist, welche die Nukleotide 114 bis 132 der SEQ ID Nr. 1 enthält, wobei in dem Oligonukleotid das dem Nukleotid 114 entsprechende Nukleotid an der ersten, zweiten oder dritten Position von dem 3'-Ende mit einem Fluoreszenzfarbstoff markiert ist;

(b) ein Oligonukleotid, welches eine Nukleotidsequenz hat, die zu der Sequenz homolog ist, welche zu einer Nukleotidsequenz mit einer Länge von 22 bis 50 Nukleotiden komplementär ist, welche die Nukleotide 123 bis 143 der SEQ ID Nr. 1 enthält, wobei in dem Oligonukleotid das dem Nukleotid 143 entsprechende Nukleotid an der ersten, zweiten oder dritten Position von dem 5'-Ende mit einem Fluoreszenzfarbstoff markiert ist;

(c) ein Oligonukleotid, welches eine Nukleotidsequenz hat, die zu einer Nukleotidsequenz homolog ist, welche eine Länge von 10 bis 50 Nukleotiden hat, welche die Nukleotide 41 bis 50 der SEQ ID Nr. 11 enthält, wobei in dem Oligonukleotid das dem Nukleotid 50 entsprechende Nukleotid an der ersten, zweiten oder dritten Position von dem 3'-Ende mit einem Fluoreszenzfarbstoff markiert ist.

2. Sonde nach Anspruch 1, bei welcher

bei dem in (a) gezeigten Oligonukleotid das dem Nukleotid 114 entsprechende Nukleotid an dem 3'-Ende mit einem Fluoreszenzfarbstoff markiert ist;

das in (b) gezeigte Oligonukleotid das dem Nukleotid 143 entsprechende Nukleotid an dem 5'-Ende hat; und/oder

das in (c) gezeigte Oligonukleotid das dem Nukleotid 50 entsprechende Nukleotid an dem 3'-Ende hat.

3. Sonde nach Anspruch 1 oder 2, bei welcher die Fluoreszenzintensität im Vergleich dazu, wenn das Oligonukleotid mit der Zielsequenz nicht hybridisiert ist, abnimmt oder zunimmt, wenn das Oligonukleotid mit der Zielsequenz hybridisiert wird.

4. Sonde nach Anspruch 3, bei welcher die Fluoreszenzintensität abnimmt, wenn das Oligonukleotid mit der Zielsequenz hybridisiert ist.

5. Sonde nach einem der Ansprüche 1 bis 4, bei welcher

das in (a) gezeigte Oligonukleotid eine Länge von 24 bis 30 Nukleotiden hat;

das in (b) gezeigte Oligonukleotid eine Länge von 29 bis 35 Nukleotiden hat; und/oder

das in (c) gezeigte Oligonukleotid eine Länge von 16 bis 22 Nukleotiden hat.

6. Sonde nach einem der Ansprüche 1 bis 4, bei welcher

das in (a) gezeigte Oligonukleotid die in SEQ ID Nr. 6 gezeigte Nukleotidsequenz hat;

das in (b) gezeigte Oligonukleotid die in SEQ ID Nr. 5 gezeigte Nukleotidsequenz hat; und/oder

das in (c) gezeigte Oligonukleotid die in SEQ ID Nr. 4 gezeigte Nukleotidsequenz hat.

7. Sonde nach einem der Ansprüche 1 bis 6, bei welcher der Polymorphismus in Exon 12 des JAK2-Gens mindestens eine Mutation ist, die ausgewählt ist aus der Gruppe bestehend aus K539L(sub1), H538QK539L(sub2), N542-E543del(del6) und F537-K539delinsL(del6inL).

8. Verfahren zum Erfassen einer Mutation in einer Nukleinsäure, welche eine polymorphe Stelle in Exon 12 des JAK2-Gens umfasst, enthaltend den Schritt der Durchführung einer Schmelzkurvenanalyse unter Verwendung einer mit einem Fluoreszenzfarbstoff markierten Nukleinsäuresonde zur Messung der Fluoreszenz von dem Fluoreszenzfarbstoff und Erfassen der Mutation auf der Grundlage des Ergebnisses der Schmelzkurvenanalyse, wobei die Nukleinsäuresonde die Sonde nach einem der Ansprüche 1 bis 7 ist.

9. Verfahren nach Anspruch 8, ferner enthaltend den Schritt des Erhaltens der Nukleinsäure, die einen Polymorphismus aufweist, durch Amplifikation der Region, die die polymorphe Stelle in der in einer Probe enthaltenen Nukleinsäure enthält.

10. Verfahren nach Anspruch 9, bei welchem die Amplifikation durch ein Verfahren, welches eine DNA-Polymerase verwendet, durchgeführt wird.

11. Verfahren nach Anspruch 10, bei welchem die Amplifikation in Anwesenheit der Sonde durchgeführt wird.

12. Sonde nach einem der Ansprüche 1 bis 7 oder Verfahren nach einem der Ansprüche 8 bis 11 zur Verwendung bei der Diagnose von chronischen myeloproliferativen Erkrankungen oder Polycythaemia vera.

**13.** Kit zum Erfassen eines Polymorphismus in Exon 12 des JAK2-Gens, enthaltend die Sonde nach einem der Ansprüche 1 bis 7.

**14.** Kit nach Anspruch 13, ferner enthaltend einen Primer zur Amplifikation der die polymorphe Stelle in Exon 12 des JAK2-Gens enthaltenden Region durch ein Verfahren, welches eine DNA-Polymerase verwendet.

**Revendications**

**1.** Sonde permettant de détecter un polymorphisme dans l'exon 12 du gène JAK2, ladite sonde comprenant au moins l'un des oligonucléotides en (a) à (c) ci-dessous :

(a) un oligonucléotide ayant une séquence nucléotidique homologue à la séquence complémentaire d'une séquence nucléotidique ayant une longueur de 19 à 50 nucléotides contenant les nucléotides 114 à 132 de SEQ ID NO : 1, où ledit oligonucléotide comporte le nucléotide correspondant au nucléotide 114 marqué par un colorant fluorescent au niveau de la première, deuxième ou troisième position à partir de l'extrémité 3' ;
(b) un oligonucléotide ayant une séquence nucléotidique homologue à la séquence complémentaire d'une séquence nucléotidique ayant une longueur de 22 à 50 nucléotides contenant les nucléotides 122 à 143 de SEQ ID NO : 1, où ledit oligonucléotide comporte le nucléotide correspondant au nucléotide 143 marqué par un colorant fluorescent au niveau de la première, seconde ou troisième position à partir de l'extrémité 5' ; et
(c) un oligonucléotide ayant une séquence nucléotidique homologue à une séquence nucléotidique ayant une longueur de 10 à 50 nucléotides contenant les nucléotides 41 à 50 de SEQ ID NO : 11, où ledit oligonucléotide comporte le nucléotide correspondant au nucléotide 50 marqué par un colorant fluorescent au niveau de la première, deuxième ou troisième position à partir de l'extrémité 3'.

**2.** Sonde selon la revendication 1, dans laquelle
ledit oligonucléotide présenté en (a) comporte le nucléotide correspondant au nucléotide 114 marqué par un colorant fluorescent à l'extrémité 3' ;
ledit oligonucléotide présenté en (b) comporte le nucléotide correspondant au nucléotide 143 à l'extrémité 5' ; et/ou
ledit oligonucléotide présenté en (c) comporte le nucléotide correspondant au nucléotide 50 à l'extrémité 3'.

**3.** Sonde selon la revendication 1 ou 2, dans laquelle l'intensité de fluorescence diminue ou augmente lorsque ledit oligonucléotide est hybridé avec la séquence cible, comparativement à lorsque ledit oligonucléotide n'est pas hybridé avec la séquence cible.

**4.** Sonde selon la revendication 3, dans laquelle l'intensité de fluorescence diminue lorsque ledit oligonucléotide est hybridé avec la séquence cible.

**5.** Sonde selon l'une quelconque des revendications 1 à 4, dans laquelle
ledit oligonucléotide présenté en (a) a une longueur de 24 à 30 nucléotides ;
ledit oligonucléotide présenté en (b) a une longueur de 29 à 35 nucléotides ; et/ou
ledit oligonucléotide présenté en (c) a une longueur de 16 à 22 nucléotides.

**6.** Sonde selon l'une quelconque des revendications 1 à 4, dans laquelle
ledit oligonucléotide présenté en (a) a la séquence nucléotidique représentée par SEQ ID NO : 6 ;
ledit oligonucléotide présenté en (b) a la séquence nucléotidique représentée par SEQ ID NO : 5 ; et/ou ledit oligonucléotide présenté en (c) a la séquence nucléotidique représentée par SEQ ID NO : 4.

**7.** Sonde selon l'une quelconque des revendications 1 à 6, où ledit polymorphisme dans l'exon 12 du gène JAK2 est au moins une mutation choisie dans le groupe constitué de K539L(sub1), H538QK539L(subs2), N542-E543del(del6) et F537-K539delinsL(del6inL).

**8.** Procédé de détection d'une mutation dans un acide nucléique comprenant un site polymorphe dans l'exon 12 du gène JAK2, comprenant l'étape de réalisation d'une analyse de la courbe de fusion en utilisant une sonde d'acide nucléique marquée par un colorant fluorescent pour la mesure de la fluorescence provenant du colorant fluorescent, et de détection de la mutation en se basant sur le résultat de l'analyse de la courbe de fusion, où la sonde d'acide nucléique est la sonde selon l'une quelconque des revendications 1 à 7.

9. Procédé selon la revendication 8, comprenant en outre l'étape d'obtention de l'acide nucléique comprenant un polymorphisme par amplification de la région contenant le site polymorphe dans l'acide nucléique contenu dans un échantillon.

10. Procédé selon la revendication 9, dans lequel l'amplification est réalisée par un procédé utilisant une ADN polymérase.

11. Procédé selon la revendication 10, dans lequel l'amplification est réalisée en présence de ladite sonde.

12. Sonde selon l'une quelconque des revendications 1 à 7 ou procédé selon l'une quelconque des revendications 8 à 11 pour une utilisation dans le diagnostic de troubles myéloprolifératifs chroniques ou d'une polycythémie vraie.

13. Kit permettant de détecter un polymorphisme dans l'exon 12 du gène JAK2, comprenant la sonde selon l'une quelconque des revendications 1 à 7.

14. Kit selon la revendication 13, comprenant en outre une amorce pour l'amplification de la région contenant le site polymorphe dans l'exon 12 du gène JAK2 par un procédé utilisant une ADN polymérase.

[Fig. 1]

(A)

(B)

[Fig. 2]

[Fig. 3]

[Fig. 4]

[Fig. 5]

[Fig. 6]

[Fig. 7]

[Fig. 8]

PROBE WT2, WAVELENGTH TAMRA

[Fig. 9]

[Fig. 10]

WHOLE BLOOD

[Fig. 11]

WT 100%

[Fig. 12]

sub1 50%

[Fig. 13]

## sub2 50%

[Fig. 14]

## del6 50%

[Fig. 15]

del6inL50%

[Fig. 16]

WT 100%

[Fig. 17]

**sub1 50%**

[Fig. 18]

sub2 50%

[Fig. 19]

del6 50%

## REFERENCES CITED IN THE DESCRIPTION

**Patent documents cited in the description**

- JP 2001286300 A
- JP 2002119291 A
- JP 2009278982 B
- WO 2007106899 A
- EP 2119796 A
- EP 1362928 A
- JP 2010105457 A

**Non-patent literature cited in the description**

- *BLOOD,* 30 July 2009, vol. 114 (5
- *Haematologica,* December 2007, vol. 92 (12), 1717-8
- *N Engl J Med.,* 01 February 2007, vol. 356 (5), 459-68
- *Haematologica,* 2009, vol. 94 (3), 414-418
- **JONES et al.** *Haematologica,* 2008, vol. 93 (10), 1560-1564
- **RAPADO et al.** *The Journal of Molecular Diagnostics,* 2009, vol. 11, 155-161
- **RADADO et al.** Annals of Hematology. 2008, vol. 87, 741-749
- **TANAKA et al.** *Leukemia research,* 2008, vol. 32, 1462-1467